# EUROPEAN PATENT SPECIFICATION

(11) **EP 3 753 503 B1**
(45) Date of publication and mention of the grant of the patent: **26.10.2022**
(21) Application number: 20180388.9
(22) Date of filing: 16.06.2020
(51) Int. Cl.: A61B 17/26, A61B 17/29, A61B 18/14

(54) **SURGICAL INSTRUMENTS FOR PERFORMING TONSILLECTOMY, ADENOIDECTOMY, AND OTHER SURGICAL PROCEDURES**
CHIRURGISCHE INSTRUMENTE ZUR DURCHFÜHRUNG VON TONSILLEKTOMIE, ADENOTOMIE UND ANDEREN CHIRURGISCHEN EINGRIFFEN
INSTRUMENTS CHIRURGICAUX PERMETTANT DE RÉALISER UNE AMYGDALECTOMIE, UNE ADÉNOÏDECTOMIE ET D'AUTRES PROCÉDURES CHIRURGICALES

(30) Priority: 17.06.2019 US 201916443192
(43) Date of publication of application: 23.12.2020
(73) Proprietor: Covidien LP, Mansfield, MA 02048 (US)
(72) Inventor: STAMM, Stephen, J, Wheat Ridge, CO 80033 (US); SAHA, Subhadeep, Boulder, CO 80301 (US); Soni, Purvishkumar, Boulder, CO 80504 (US); Olson, Jessica, Frederick, CO 80504 (US); Sawyer, Alyssa, Broomfield, CO 80020 (US)
(74) Representative: Maschio, Antonio

(56) References cited:
- EP-A2- 3 095 399
- WO-A1-2018/131039
- DE-A1-102009 043 471
- US-A- 4 901 708
- US-A1- 2006 041 274
- US-A1- 2006 079 924
- US-A1- 2014 114 293
- US-A1- 2017 360 465

## Description

### BACKGROUND

### Technical Field

The present disclosure relates to surgical instruments and, more particularly, to surgical instruments for performing tonsillectomy, adenoidectomy, and other surgical procedures.

### Background of Related Art

The tonsils and adenoids are part of the lymphatic system and are generally located in the back of the throat. These parts of the lymphatic system are generally used for sampling bacteria and viruses entering the body and activating the immune system when warranted to produce antibodies to fight oncoming infections. More particularly, the tonsils and adenoids break down the bacteria or virus and send pieces of the bacteria or virus to the immune system to produce antibodies for fighting off infections.

Inflammation of the tonsils and adenoids (e.g., tonsillitis) impedes the ability of the tonsils and adenoids to destroy the bacteria resulting in a bacterial infection. In many instances, the bacteria remain even after treatment and serve as a reservoir for repeated infections (e.g., tonsillitis or ear infections).

A tonsillectomy and/or adenoidectomy may be performed when infections persist and antibiotic treatments fail. Some individuals are also born with larger tonsils that are more prone to cause obstruction. An adenoidectomy may also be required to remove adenoid tissue when ear pain persists, or when nose breathing or function of the Eustachian tube is impaired. Often times, tonsillectomy and adenoidectomy procedures are performed at the same time.

EP 3095399 A1 describes surgical instruments for adenoidectomy or tonsillectomy according the preamble of claim 1.

US 2006/079924 A1 describes surgical apparatus for expanding a surgical cavity, in particular a speculum for the vaginal cavity. The apparatus comprises an inflatable body, an insertion sheath, and an expandable support assembly. A concave channel may be provided on an inward-facing surface of the support assembly to provide an unobstructed view through the lumen thereof.

US 4901708 A describes a laryngoscope having a handle member attached to a curved blade member. The curved blade member includes a spatula member having a concave channel running along its length to allow a medical professional to have a greater line of sight view along the blade member.

### SUMMARY

As used herein, the term "distal" refers to the portion that is being described which is further from a user, while the term "proximal" refers to the portion that is being described which is closer to a user. Further, to the extent consistent, any of the aspects described herein may be used in conjunction with any or all of the other aspects described herein.

A surgical instrument provided in accordance with the present invention includes a housing having a barrel with distal and proximal end portions. The barrel includes a handle depending from a lower end thereof and a concave portion defined within and extending along an upper surface thereof from the proximal end portion of the barrel to the distal end portion of the barrel. A shaft is included and extends from the distal end portion of the barrel and supports an end effector assembly at a distal end thereof configured to treat tissue. The concave portion enhances visibility of the end effector assembly during tissue treatment.

In embodiments of the present invention, the surgical instrument further includes a movable handle operably coupled to the housing and movable relative thereto between an initial position and a compressed position. In embodiments according to the present invention, a trigger is operably coupled to the housing within a slot defined therein, the trigger movable relative to the housing and the movable handle between an un-actuated position and an actuated position. In such embodiments, movement of the trigger from the un-actuated position to the actuated position suitably deploys a knife.

In such embodiments according to the present invention, the movable handle and the trigger are pivotably coupled to the housing. In yet other embodiments according to the present invention, the movable handle and the trigger are pivotably coupled to the housing about a common pivot. In still other embodiments according to the present invention, the trigger at least partially surrounds the movable handle.

In embodiments according to the present invention, the barrel includes a width and the concave portion extends at least partially across the width of the barrel. In other embodiments according to the present invention, the barrel includes a width and the concave portion extends fully across the width of the barrel.

### BRIEF DESCRIPTION OF THE DRAWINGS

Various aspects and features of the present invention are described herein with reference to the drawings wherein:
FIG. 1 is a perspective view of a reference surgical instrument not in accordance with the present invention including a handle assembly, end effector assembly, trigger assembly and activation assembly; with jaw members of the end effector assembly of the surgical instrument disposed in a spaced-apart position;
FIGS. 2A-2D are rear, perspective, internal views of the surgical instrument of FIG. 1 showing the range of movement of a pair of jaw members of the end effector assembly and the movement of the various internal components of the handle assembly, trigger assembly and activation assembly;
FIG. 3 is a front, perspective, partially-exploded view of the surgical instrument of FIG. 1 with the jaw members disposed in the approximated position and a portion of the housing removed to illustrate the internal components thereof;
FIGS. 4A-4C are enlarged, perspective views of the jaw members of the end effector assembly shown in open and approximated positions and showing advancement of a knife blade to cut tissue disposed between jaw members;
FIG. 5 is rear, perspective view of an embodiment of the surgical instrument in accordance with the present invention;
FIG. 6 is rear, perspective view of yet another surgical instrument not in accordance with the present invention; and
FIG. 7 is an enlarged, perspective view of an end effector assembly of another surgical instrument not in accordance with the present invention.

### DETAILED DESCRIPTION

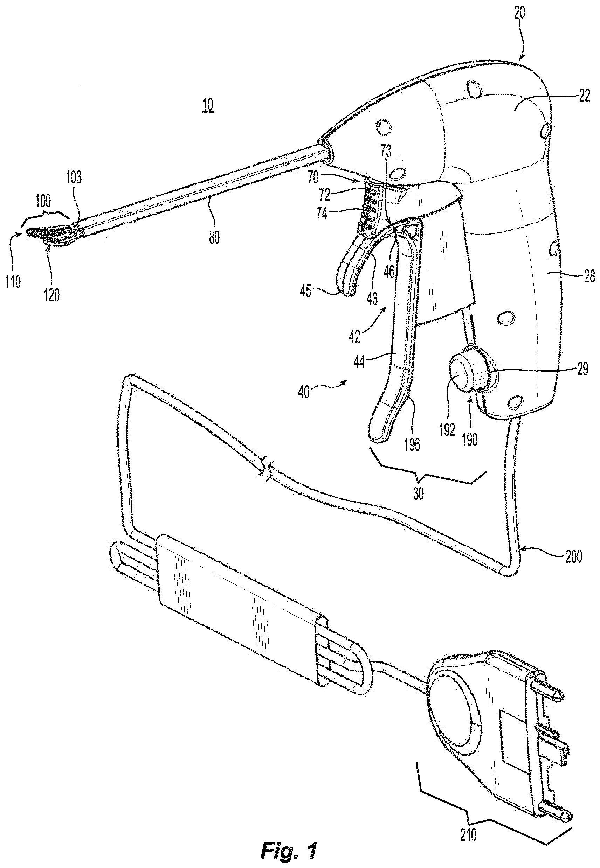

Referring generally to FIG. 1, the reference surgical instrument is shown generally identified by reference numeral 10. Instrument 10, as described below, is configured for grasping, treating, and/or dissecting tissue and may find particular applicability for use in performing tonsillectomy procedures and/or adenoidectomy procedures, although use of instrument 10 in various other surgical procedures is also contemplated. Additional features contemplated for use with instrument 10 are detailed in commonly-owned U.S. Patent Applications US 2016/338762 A1, US 2016/338718 A1, US 2016/338719 A1, and US 2016/338764 A1, each of which was filed on May 22, 2015.

With reference to FIGS, 1-3, instrument 10 generally includes a housing 20, a handle assembly 30, a trigger assembly 70, a shaft 80, an end effector assembly 100, a drive assembly 140, a knife assembly 170, and an energy activation assembly 190. As detailed below, shaft 80 extends distally from housing 20 and supports end effector assembly 100 at distal end of shaft 80, drive assembly 140 operably couples handle assembly 30 with end effector assembly 100 to enable selective manipulation of jaw members 110, 120 of end effector assembly 100, knife assembly 170 is operably coupled with trigger assembly 70 to enable selective translation of a knife blade 174 of knife assembly 170 relative to end effector assembly 100, and energy activation assembly 190 enables energy to be selectively delivered to end effector assembly 100.

Instrument 10 also includes an electrosurgical cable 200 including a proximal plug 210 that connects instrument 10 to a generator (not shown) or other suitable power source, although instrument 10 may alternatively be configured as a battery-powered instrument. Electrosurgical cable 200 includes lead wires extending therethrough that have sufficient length to extend through housing 20 and shaft 80 in order to operably couple the generator, energy activation assembly 190, and end effector assembly 100 with one another to enable the selective supply of energy to jaw members 110, 120 of end effector assembly 100, e.g., upon activation of activation switch 194 of energy activation assembly 190.

Housing 20 houses the internal working components of instrument 10 and is formed from first and second housing components configured to engage one another via a plurality of pin-aperture engagements spaced around housing 20, although other suitable engagements, e.g., screws, snap-fit connections, adhesion, ultrasonic welding, etc., are also contemplated, as are different formations of housing 20. Housing 20 defines a pistol-style configuration having a longitudinally-extending barrel portion 22 and a fixed handle portion 28 that extends from barrel portion 22 in generally perpendicular orientation relative thereto.

Barrel portion 22 of housing 20 defines a distal aperture configured to receive and engage the proximal end of shaft 80 therein. Shaft 80 extends distally from barrel portion 22 of housing 20 and defines a generally rectangular cross-sectional configuration oriented such that the larger width dimension thereof extends laterally and the smaller height dimension thereof extends vertically. This configuration of shaft 80 relative to the orientation of jaw members 110, 120 provides enhanced "line-of-sight" for visualizing the surgical site adjacent end effector assembly 100. As described in greater detail below, shaft 80 includes a pair of spaced-apart clevis members 84 extending from the top and bottom walls, e.g., the larger width dimension walls, of shaft 80 at the distal end of shaft 80. Each clevis member 84 defines an aperture for receiving a pivot pin 103 to operably support end effector assembly 100 at the distal end of shaft 80.

Barrel portion 22 of housing further includes a pair of opposed pivot apertures 23 (only one of which is shown), a longitudinal track 24, a pair of opposed pivot bosses 25 (only one of which is shown), and a block 26. Each pivot aperture 23 is configured to receive an end of pivot pin 48 to pivotably couple movable handle 40 and trigger 72 to housing 20. Longitudinal track 24 is configured to guide translation of drive assembly 140 relative to housing 20. Pivot bosses 25 extend inwardly into housing 20 and are configured to pivotably couple linkage 76 of trigger assembly 70 to housing 20..

Energy activation assembly 190 includes a depressible button 192 that is mechanically coupled to a switch 194 mounted within a bay 29 defined within fixed handle portion 28 of housing 20 and is engagable by a button activation post 196 extending proximally from a proximal side of movable handle 40 upon movement of movable handle 40 to the activated position, as detailed below. Switch 194 is configured to electrically communicate with end effector assembly 100 and the generator (not shown) via suitable electrical wiring to enable energy to be supplied from the generator (not shown) to end effector assembly 100 upon activation of switch 194.

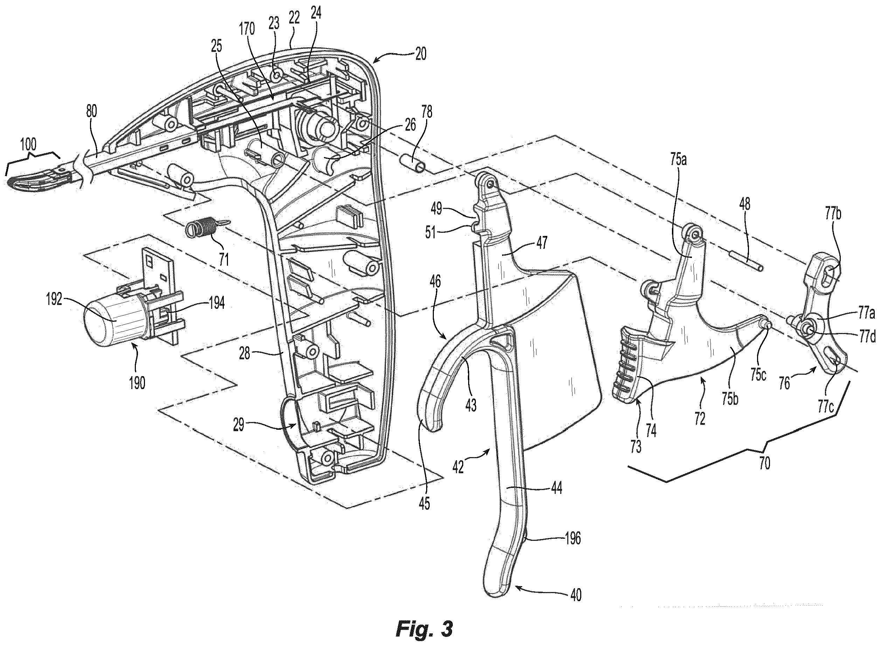

Continuing with reference to FIGS. 1-3, handle assembly 30 includes movable handle 40 that is movable relative to fixed handle portion 28 of housing 20 between an initial position, a compressed position, and an activated position, as explained in greater detail below, to impart movement of jaw members 110, 120 of end effector assembly 100 between a spaced-apart position and an approximated position for grasping tissue therebetween and for initiating the supply of energy to end effector assembly 100 for treating grasped tissue. Movable handle 40 and trigger 72 of trigger assembly 70 are ergonomically configured to facilitate manipulation and operation of instrument 10. Movable handle 40, more specifically, defines a grasping portion 42 having an arcuate segment 43 and an elongated proximal leg 44 that extends from arcuate segment 43 the length of fixed handle portion 28 of housing 20. Arcuate segment 43 culminates in a distal tail 45 and defines a sufficient diameter so as to operably receive a user's finger between distal tail 45 and proximal leg 44. Arcuate segment 43 further defines a convex surface 46. Trigger 72, more specifically, defines an abutting surface 73 that abuts convex surface 46 of arcuate segment 43 of movable handle 40 and is complementarily contoured such that, in the initial position of movable handle 40 and the un-actuated position of trigger 72, pinch points between trigger 72 and movable handle 40 are eliminated. Further, trigger 72 surrounds the exposed part of flange portion 47 of movable handle 40 to eliminate pinch points therebetween.

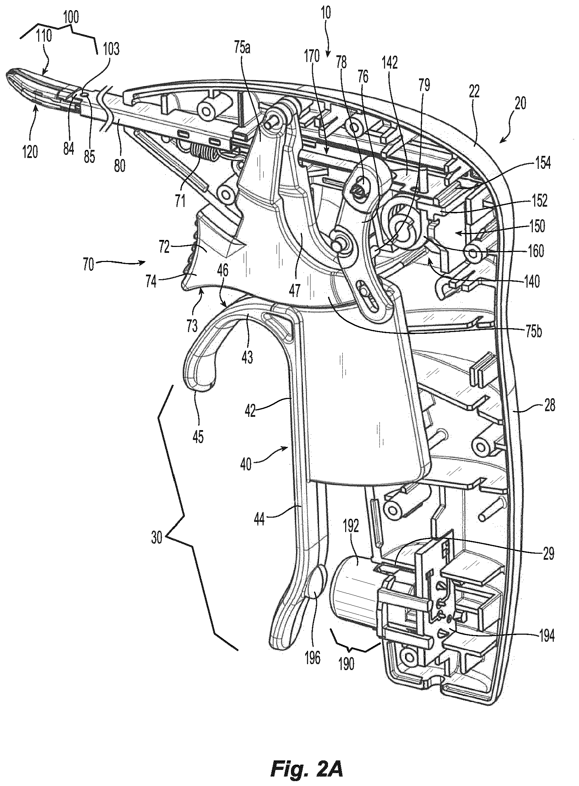

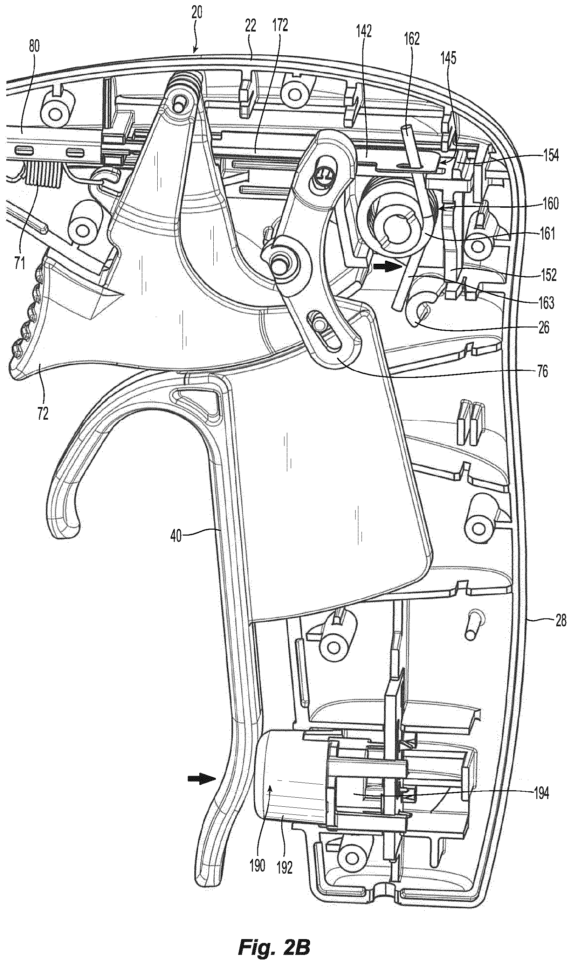

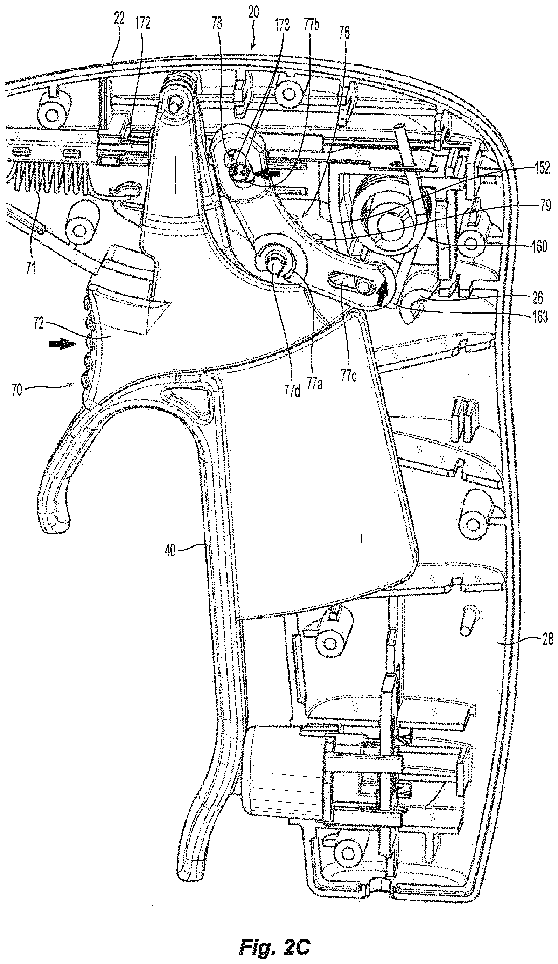

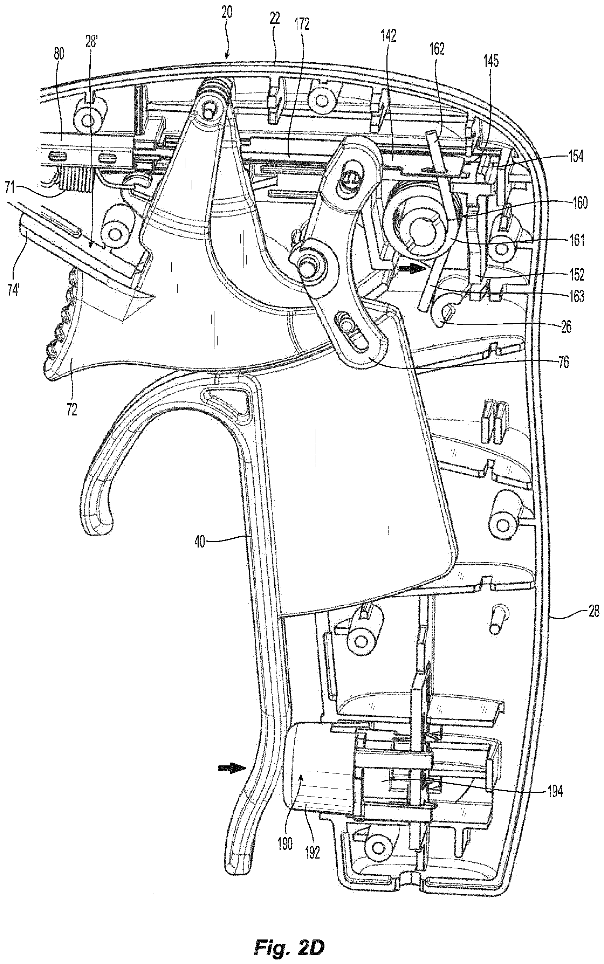

Movable handle 40, as noted above, includes grasping portion 42, which extends from housing 20 adjacent fixed handle portion 28, and flange portion 47, which extends upwardly into housing 20. Flange portion 47 is pivotably coupled within housing 20 at the free end of flange portion 47 via pivot pin 48. Pivot pin 48 is engaged within and extends between pivot apertures 23 of housing 20 to permit movable handle 40 to pivot about pivot pin 48 and relative to housing 20 between the initial position (FIGS. 1 and 2), the compressed position, and the activated position.

In use movable handle 40 is biased towards the initial position by the abutment of a lower leg 163 of a drive torsion spring (not shown) with block 26 of housing 20. With movable handle 40 in the initial position, slider assembly 150 is likewise disposed in a distal-most position. With slider assembly 150 disposed in its distal-most position, an upper leg 162 of the drive torsion spring 160 retains drive plate 142 in a distal-most position with the proximal edge 145 of drive plate 142 disposed in abutment with abutment rib 154 of proximal housing 152 of slider assembly 150. In the distal-most position of drive plate 142, drive plate 142 maintains the jaw cam pin (not shown) at the distal ends of oppositely-angled cam slots of the proximal flanges of the jaw members 110, 120 to thereby maintain jaw members 110, 120 in the spaced-apart position.

At this point, trigger 72 is disposed in the un-actuated position, wherein trigger 72 is in a distal-most position under the bias of biasing member 71 such that upper end cam slot 77b of linkage 76 is disposed in a proximal-most position while lower end cam slot 77c of linkage 76 is disposed in a distal-most position. Thus, knife plate 172 is disposed in a proximal-most position, corresponding to a retracted position of knife blade 174, wherein knife blade 174 is disposed between proximal flanges of jaw frames of jaw members 110, 120 but does not extend distally therefrom. Further, with movable handle 40 disposed in its initial position, proximal housing 152 of slider assembly 150 is disposed in the movement path of lockout peg 79 of linkage 76, inhibiting rotation of linkage 76 and, thus, inhibiting movement of trigger 72 from the un-actuated position to the actuated position. As such, knife blade 174 is inhibited from being deployed when jaw members 110, 120 are disposed in the spaced-apart position.

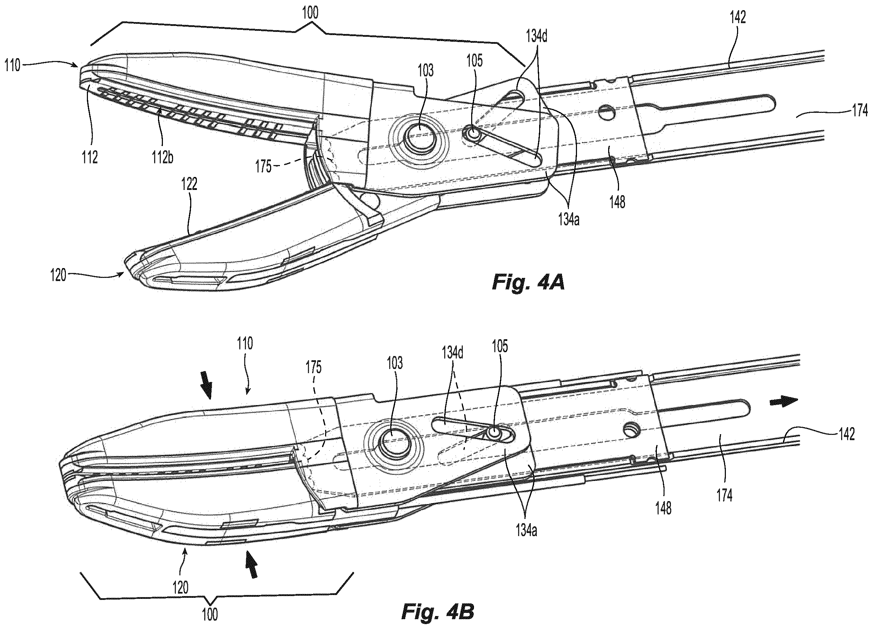

In order to move jaw members 110, 120 to the approximated position to grasp tissue therebetween, movable handle 40 is pulled proximally towards fixed handle portion 28 of housing 20 from the initial position to the compressed position (FIGS. 2A and 2B). Upon movement of movable handle 40 to the compressed position, movable handle 40 urges slider assembly 150 proximally through housing 20. Torsion spring 160, in an initial, less-tensioned state, is translated proximally together with slider assembly 150 such that upper leg 162 of torsion spring 160 pulls drive plate 142 proximally in connection with the proximal translation of slider assembly 150. In other words, at this point, slider assembly 150 and drive plate 142 move in concert with one another. As drive plate 142 is pulled proximally, cam pin 105 is pulled proximally through cam slots 134d of proximal flanges 134a of jaw members 110, 120 such that jaw members 110, 120 are pivoted from the spaced-apart position to the approximated position (FIGS. 4A and 4B) to grasp tissue therebetween.

In order to apply energy to tissue grasped between jaw members 110, 120 to treat tissue, movable handle 40 is compressed further towards fixed handle portion 28 of housing 20 to an activation position, wherein an appropriate closure force or closure force within an appropriate range, is achieved and energy activation is initiated (See FIG. 2D). As movable handle 40 is moved further proximally relative to housing 20 beyond the compressed position, an appropriate closure force or closure force within an appropriate range is imparted to tissue grasped between jaw members 110, 120 regardless of the thickness or compressibility of tissue or the position of movable handle 40. This is because, upon movement of movable handle 40 from the compressed position towards the activation position, proximal housing 152 of slider assembly 150 is translated proximally while drive plate 142 is maintained in position. In other words, upon movement of movable handle 40 from the compressed position to the activated position, proximal housing 152 and drive plate 142 no longer move in concert with one another but are decoupled to permit relative motion therebetween.

The decoupling of proximal housing 152 of slider assembly 150 and drive plate 142 to permit relative motion therebetween is provided via torsion spring 160. More specifically, upon proximal movement of movable handle 40, a first force is imparted from movable handle 40, through proximal housing 152 of slider assembly 150, body 161 of torsion spring 160, and upper leg 162 of torsion spring 160, to drive plate 142 to urge drive plate 142 in a proximal direction, while a second, opposite force acts on drive plate 142 and, thus, upper leg 162 of torsion spring 160 in a distal direction to control the amount of compression of tissue between jaw members 110, 120. Once the second, opposite force exceeds the spring force of torsion spring 160, proximal movement of proximal housing 152 no longer results in proximal movement of drive plate 142 but, rather, results in further tensioning of torsion spring 160, wherein torsion spring 160 is wound-up, absorbing the force imparted thereto from movement of movable handle 40.

Thus, once this point has been reached, further proximal translation of proximal housing 152 of slider assembly 150 urges body 161 of torsion spring 160 proximally while upper leg 162 of torsion spring 160 remains in position as a result of the wind-up tensioning of torsion spring 160. With upper leg 162 of torsion spring 160 retained in position, drive plate 142 is likewise retained in position despite the proximal translation of movable handle 40. As such, an upper threshold of pressure applied to tissue grasped between jaw members 110, 120 is defined.

Referring to FIG. 2D, upon achieving the activation position of movable handle 40, button activation post 196 (FIG. 1) of movable handle 40 contacts depressible button 192 sufficiently so as to depress depressible button 192 into fixed handle portion 28 of housing 20 to activate switch 194. Switch 194, as noted above, is disposed in electrical communication with the generator (not shown) and electrically-conductive plates 112, 122 of jaw members 110, 120 (FIG. 4A), such that activation of switch 194 initiates the supply of energy to electrically-conductive plates 112, 122 to treat, e.g., coagulate, cauterize, and/or seal, tissue grasped therebetween.

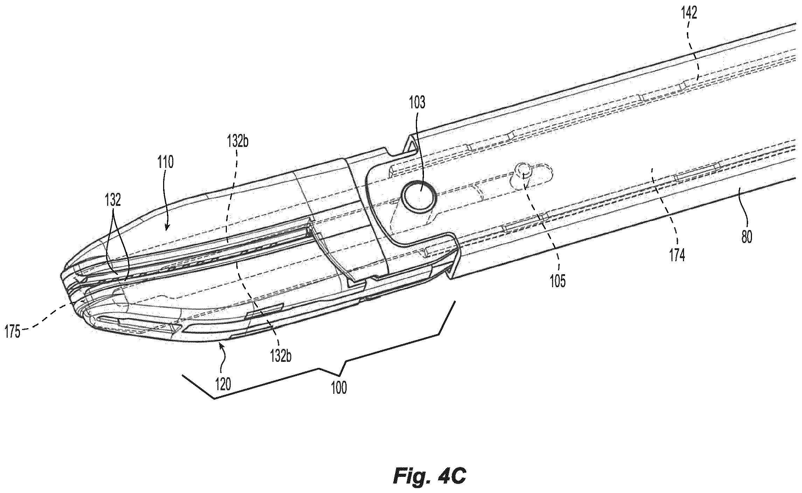

Referring to FIG. 4C, once tissue has been treated or where it is only desired to cut tissue, knife blade 174 may be advanced between jaw members 110, 120 to cut tissue grasped therebetween. In order to advance knife blade 174 from the retracted position to the extended position, trigger 72 is pulled proximally against the bias of biasing member 71 from the un-actuated position to the actuated position (FIG. 2C). As trigger 72 is pulled proximally, linkage 76 is urged to pivot counter-clockwise (compare FIG. 2C to FIG. 2D) such that upper end slot 77b of linkage 76 is moved distally. Distal movement of upper end slot 77b urges tube 78 to translate distally and, in turn, urges knife plate 172 to translate distally. This movement is permitted as proximal housing 152 is displaced relative to the movement path of lockout peg 79 with movable handle 40 in or near the compressed or actuated position.

As detailed above, movement of trigger 72 from the un-actuated position to the actuated position urges knife plate 172 distally. More specifically, knife plate 172 is urged distally such that knife blade 174 is advanced distally from the retracted position to the extended position. As knife blade 174 is advanced distally, knife blade 174 extends through knife slots 112a, 112b defined within electrically-conductive plates 112, 122 to cut tissue grasped between jaw members 110, 120.

Upon release, trigger 72 and knife plate 172 are returned proximally under the bias of biasing member 71 such that knife blade 174 is returned to the retracted position. Thereafter, movable handle 40 may be released, allowing movable handle 40 to return to the initial position under the bias of lower leg 163 of torsion spring 160 abutting block 26 of housing 20, thereby returning jaw members 110, 120 to the spaced-apart position and releasing the treated and/or divided tissue.

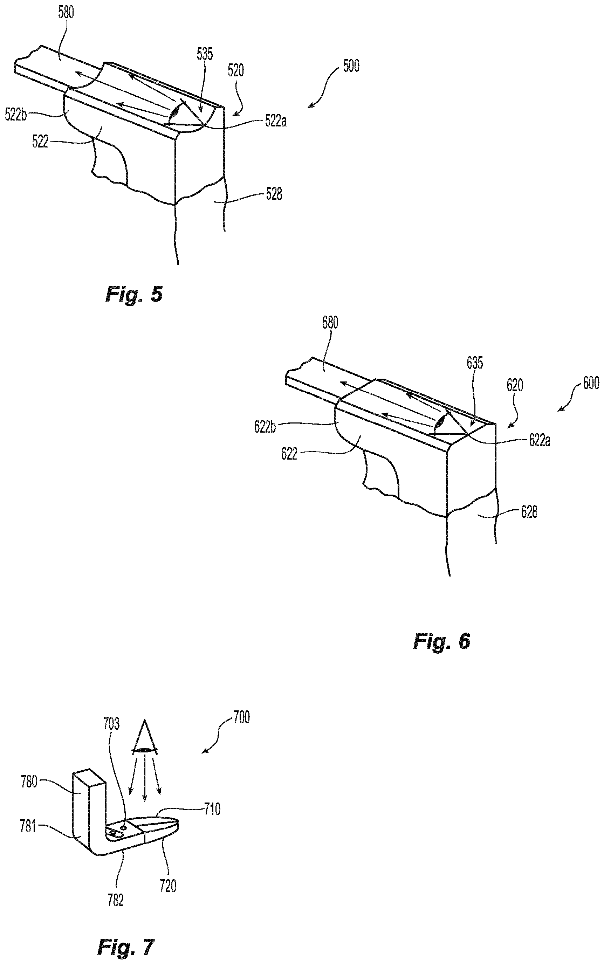

FIG. 5 shows an embodiment of the surgical instrument 500 in accordance with the present invention. Instrument 500 includes many of the same elements as the aforedescribed reference surgical instrument 10 and only the differences will be described herein for the purposes of brevity. Instrument 500 incudes a housing 520 including a barrel portion 522 and an integral handle 528 depending therefrom. Similar to instrument 10, a shaft 580 extends from a distal portion 522b of barrel portion 522 for ultimate connection to end effector assembly 100 (as described above).

Barrel portion 522 includes a concave portion 535 extending along an upper surface thereof from the proximal portion 522a of the barrel portion 522 to the distal portion 522a of the barrel portion 522. The concave portion 535 is configured to enhance line-of-sight to the end effector assembly 100 during surgery and handling. The concave portion 535 may be dimensioned at varying depths depending upon a particular purpose or a particular surgical need. In embodiments, the concave portion 535 extends the entire width of the barrel portion 522 to enhance line-of-sight for the surgeon. In other embodiments, the concave portion 535 extends partially across the width of the barrel portion 522. Other instruments are envisioned that may provide a maximum concave portion.

FIG. 6 shows another reference surgical instrument 600 for use with various surgical procedures. Instrument 600 includes many of the same elements as the aforedescribed surgical instrument 10 and only the differences will be described herein for the purposes of brevity. Instrument 600 incudes a housing 620 including a barrel portion 622 and an integral handle 628 depending therefrom. Similar to instrument 10, a shaft 680 extends from a distal portion 622b of barrel portion 622 for ultimate connection to end effector assembly 100 (as described above).

Barrel portion 622 includes a substantially flat portion 635 extending along an upper surface thereof from the proximal portion 622a of the barrel portion 622 to the distal portion 622a of the barrel portion 622. The flat portion 635 is configured to enhance line-of-sight to the end effector assembly 100 during surgery and handling. The flat portion 635 extends the entire width of the barrel portion 622 to enhance line-of-sight for the surgeon. In other embodiments, the flat portion 635 may extend partially across the width of the barrel portion 622 and be configured more like a notch.

FIG. 7 shows another referece end effector assembly 700 for use with various surgical procedures. End effector assembly 700 includes many of the same elements as the aforedescribed end effector assembly 100 and surgical instrument 10 and only the differences will be described herein for the purposes of brevity. End effector assembly 700 incudes first and second jaw members 710 and 720, respectively, which operative engage a distal end 782 of shaft 780. First and second jaw members 710, 720 are moveable about a pivot 703 relative to one another (or one jaw member, e.g., jaw members 710, is movable relative to the other jaw member, e.g., jaw member 120) from a first spaced position relative to one another to a position closer to one another for treating or grasping tissue.

Distal end 782 of the end effector assembly 700 is angled at elbow 781 such that the end effector assembly 700 extends at an angle of about ninety degrees (90°) relative to a longitudinal axis of the shaft 780. Orienting the distal end 782 at an angle of about 90° relative to the proximal end of the shaft 780 provides better line-of-sight of the end effector assembly 700 for the surgeon during manipulation and handling. The distal end 782 may be configured at angles other than 90° (less than 90° or greater than 90°) to facilitate a particular purpose or to achieve a particular result, e.g., better line-of-sight of the end effector assembly 700 during a particular surgery wherein the view of the end effector assembly 700 may be obstructed or otherwise compromised.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

From the foregoing and with reference to the various figure drawings, those skilled in the art will appreciate that certain modifications can also be made to the embodiments shown in the drawings, within the scope of the claims appended hereto. The invention is defined by the following claims.

## Claims

1. A surgical instrument (500), comprising:
a housing (520) having a barrel (522) with distal and proximal end portions (522a, 522b), the barrel including a handle (528) depending from a lower end thereof; and
a shaft (580) extending from the distal end portion of the barrel (522), the shaft supporting an end effector assembly (100) at a distal end thereof configured to treat tissue,
**characterized in that** the barrel (522) includes a concave portion (535) defined within and extending along an upper surface thereof from the proximal end portion (522a) of the barrel to the distal end portion (522b) of the barrel, wherein the concave portion (535) is configured to enhance visibility of the end effector assembly (100) during tissue treatment.

2. The surgical instrument according to claim 1, further comprising:
a movable handle (40) operably coupled to the housing (520) and movable relative thereto between an initial position and a compressed position.

3. The surgical instrument according to claim 2, further comprising:
a trigger (72) operably coupled to the housing (520) within a slot defined therein, the trigger movable relative to the housing and the movable handle (40) between an un-actuated position and an actuated position.

4. The surgical instrument according to claim 3, wherein movement of the trigger (72) from the un-actuated position to the actuated position deploys a knife (174).

5. The surgical instrument according to claim 3, wherein the movable handle (40) and the trigger (72) are pivotably coupled to the housing (520).

6. The surgical instrument according to claim 5, wherein the movable handle (40) and the trigger (72) are pivotably coupled to the housing (520) about a common pivot (48).

7. The surgical instrument according to claim 3, wherein the trigger (72) at least partially surrounds the movable handle (40).

8. The surgical instrument according to any of claims 1 to 7, wherein the barrel (522) includes a width and the concave portion (535) extends at least partially across the width of the barrel.

9. The surgical instrument according to any of claims 1 to 7, wherein the barrel (522) includes a width and the concave portion (535) extends fully across the width of the barrel.

## Patentansprüche

1. Chirurgisches Instrument (500), umfassend:
ein Gehäuse (520), das einen Zylinder (522) mit einem distalen und einem proximalen Endabschnitt (522a, 522b) aufweist, wobei der Zylinder einen Griff (528) beinhaltet, der von einem unteren Ende davon abhängt; und
einen Schaft (580), der sich von dem distalen Endabschnitt des Zylinders (522) erstreckt, wobei der Schaft eine Endeffektoranordnung (100) an einem distalen Ende davon trägt, die konfiguriert ist, um Gewebe zu behandeln,
**dadurch gekennzeichnet, dass** der Zylinder (522) einen konkaven Abschnitt (535) beinhaltet, der innerhalb einer oberen Oberfläche davon von dem proximalen Endabschnitt (522a) des Zylinders zu dem distalen Endabschnitt (522b) des Zylinders definiert ist und sich entlang dieser erstreckt, wobei der konkave Abschnitt (535) konfiguriert ist, um eine Sichtbarkeit der Endeffektoranordnung (100) während der Gewebebehandlung zu verbessern.

2. Chirurgisches Instrument nach Anspruch 1, ferner umfassend:
einen bewegbaren Griff (40), der mit dem Gehäuse (520) wirkgekoppelt ist und relativ dazu zwischen einer initialen Position und einer komprimierten Position bewegbar ist.

3. Chirurgisches Instrument nach Anspruch 2, ferner umfassend:
einen Auslöser (72), der mit dem Gehäuse (520) innerhalb eines darin definierten Schlitzes wirkgekoppelt ist, wobei der Auslöser relativ zu dem Gehäuse und dem bewegbaren Griff (40) zwischen einer nicht betätigten Position und einer betätigten Position bewegbar ist.

4. Chirurgisches Instrument nach Anspruch 3, wobei die Bewegung des Auslösers (72) von der nicht betätigten Position in die betätigte Position ein Messer (174) entfaltet.

5. Chirurgisches Instrument nach Anspruch 3, wobei der bewegbare Griff (40) und der Auslöser (72) mit dem Gehäuse (520) drehbar gekoppelt sind.

6. Chirurgisches Instrument nach Anspruch 5, wobei der bewegbare Griff (40) und der Auslöser (72) mit dem Gehäuse (520) um einen gemeinsamen Drehpunkt (48) drehbar gekoppelt sind.

7. Chirurgisches Instrument nach Anspruch 3, wobei der Auslöser (72) den bewegbaren Griff (40) mindestens teilweise umgibt.

8. Chirurgisches Instrument nach einem der Ansprüche 1 bis 7, wobei der Zylinder (522) eine Breite beinhaltet und sich der konkave Abschnitt (535) mindestens teilweise über die Breite des Zylinders hinweg erstreckt.

9. Chirurgisches Instrument nach einem der Ansprüche 1 bis 7, wobei der Zylinder (522) eine Breite beinhaltet und sich der konkave Abschnitt (535) vollständig über die Breite des Zylinders hinweg erstreckt.

## Revendications

1. Instrument chirurgical (500), comprenant :
un boîtier (520) ayant un cylindre (522) avec des parties d'extrémité distale et proximale (522a, 522b), le cylindre comportant une poignée (528) dépendant d'une extrémité inférieure de celui-ci ; et
une tige (580) s'étendant à partir de la partie d'extrémité distale du cylindre (522), la tige supportant un ensemble effecteur d'extrémité (100) à une extrémité distale de celui-ci conçue pour traiter le tissu,
**caractérisé en ce que** le cylindre (522) comporte une partie concave (535) définie à l'intérieur et s'étendant le long d'une surface supérieure de celui-ci de la partie d'extrémité proximale (522a) du cylindre à la partie d'extrémité distale (522b) du cylindre, la partie concave (535) étant conçue pour améliorer la visibilité de l'ensemble effecteur d'extrémité (100) pendant le traitement du tissu.

2. Instrument chirurgical selon la revendication 1, comprenant en outre :
une poignée mobile (40) accouplée de manière fonctionnelle au boîtier (520) et mobile par rapport à celui-ci entre une position initiale et une position comprimée.

3. Instrument chirurgical selon la revendication 2, comprenant en outre :
une détente (72) accouplée de manière fonctionnelle au boîtier (520) à l'intérieur d'une fente définie à l'intérieur, la détente étant mobile par rapport au boîtier et la poignée mobile (40) entre une position non actionnée et une position actionnée.

4. Instrument chirurgical selon la revendication 3, dans lequel le mouvement de la détente (72) de la position non actionnée à la position actionnée déploie un couteau (174).

5. Instrument chirurgical selon la revendication 3, dans lequel la poignée mobile (40) et la détente (72) sont accouplées de manière pivotante au boîtier (520).

6. Instrument chirurgical selon la revendication 5, dans lequel la poignée mobile (40) et la détente (72) sont accouplées de manière pivotante au boîtier (520) par rapport au pivot commun (48).

7. Instrument chirurgical selon la revendication 3, dans lequel la détente (72) entoure au moins partiellement la poignée mobile (40).

8. Instrument chirurgical selon l'une quelconque des revendications 1 à 7, dans lequel le cylindre (522) comporte une largeur et la partie concave (535) s'étend au moins partiellement sur la largeur du cylindre.

9. Instrument chirurgical selon l'une quelconque des revendications 1 à 7, dans lequel le cylindre (522) comporte une largeur et la partie concave (535) s'étend entièrement sur la largeur du cylindre.
